# EUROPEAN PATENT SPECIFICATION

(11) **EP 0 845 220 B1**
(45) Date of publication and mention of the grant of the patent: **03.09.2003**
(21) Application number: 97925295.4
(22) Date of filing: 09.06.1997
(51) Int. Cl.: A24F 47/00, A61M 15/06

(54) **FLAVOR PRODUCING ARTICLE**
AROMAERZEUGENDER ARTIKEL
PARFUMEUR D'AMBIANCE

(30) Priority: 17.06.1996 JP 15563696
(43) Date of publication of application: 03.06.1998
(73) Proprietor: Japan Tobacco Inc., Minato-Ku Tokyo 105 (JP)
(72) Inventor: SUSA, Masayuki-Japan Tobacco Inc., Tokyo 130 (JP); TAKEUCHI, Manabu-Japan Tobacco Inc., Tokyo 130 (JP); KOBAYASHI, Takeshi-Japan Tobacco Inc. Engin., Kanagawa-ken 254 (JP); SASAKI, Hiroshi-Japan Tobacco Inc. Eng. R+DCenterD, Kanagawa-ken254 n (JP); BANDAI, Takeshi-Japan Tobacco Inc. Eng. R+DCenterD, Kanagawa-ken254 n (JP)
(74) Representative: Ruffles, Graham Keith
(86) International application number: JP9701953
(87) International publication number: WO97048293

(56) References cited:
- WO-A-95/01137
- JP-A- 2 124 081
- JP-A- 2 124 082
- JP-B- 48 008 231
- US-A- 4 303 083
- US-A- 4 735 217
- US-A- 4 846 199

## Description

### Technical Field

The present invention relates to a flavor generation article employed for enjoying inhalation of a flavor and simulated smoking and, more particularly, to a flavor generation article used for generating the flavor as an inhalation target by heating a liquid material with an electric heater.

### Background Art

A simulated smoking article employed for enjoying the flavor and smoke of tobacco without burning tobacco is already known, and various types of simulated smoking articles have been proposed.

Jpn. Pat. Appln. KOKAI Publication No. 3-232481 discloses a typical concept of a conventional simulated smoking article. The article of this reference uses, e.g., a rod-like solid material. When the solid material is heated by a heating element, an inhalation target, e.g., a flavor, is generated. The drawbacks of the article of this type are as follows. When the solid material is continuously heated, the material is largely wasted. Inversely, when the solid material is heated in accordance with inhalation of the user, a large time lag occurs between the start of inhalation (one puffing operation) of the user and generation of the flavor.

As an example of an article that copes with the above drawbacks, Jpn. Pat. Appln. KOKAI Publication No. 3-277265 discloses a flavor emitting article having a solid material divided into a large number of portions. In the article of this reference, the respective portions of the solid material are sequentially heated in units of puffing operations of the user to generate an inhalation target, e.g., a flavor or the like. The drawback of this article is that the solid material and a heating element constitute an integral flavor generation medium. Therefore, when the material is consumed, the heating element must be exchanged or disposed of together with the material, which is not preferable both in terms of economy and environment.

Jpn. Pat. Appln. KOKAI Publication No. 5-212100 discloses an example of a mechanism that detects one puffing operation of the user. In the article of this reference, the driving operation of a heating element for heating the flavor material is controlled by a signal obtained from the motion of the lips of the user.

U.S.P. No. 4,945,931 discloses a simulated smoking article using a pressurized aerosol container. In the article of this reference, the puffing operation of the user swings the vanes to mechanically open the outlet port of the container, and the aerosol is emitted. As a modification, this reference also discloses an article in which a heating element for heating aerosol cooled by the heat of evaporation is disposed in the outlet port of the container. The drawback of this reference is as follows. Since the pressurized aerosol is closed in the container with a valve which is opened/closed merely by the puffing operation of the user, once the valve is opened, a large amount of aerosol leaks undesirably. More specifically, in the article of this reference, a predetermined amount of aerosol appropriate for one puffing operation cannot be continuously emitted, and rather all of the pressurized flavor gas may undesirably be emitted until a puffing operation is complete twice or three times.

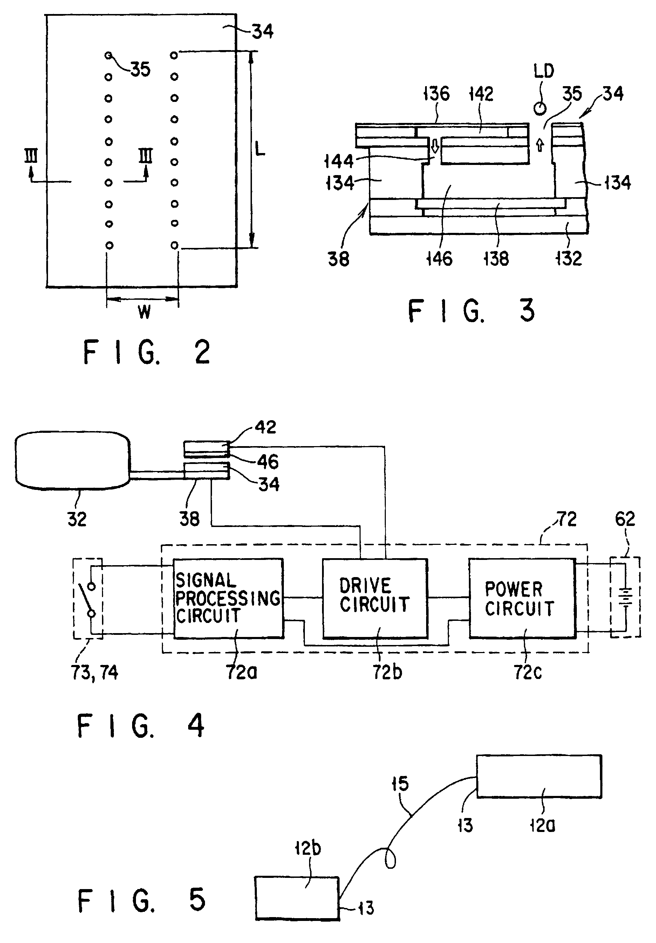

WO 9501137 provides a dispenser which comprises a reservoir of a physiologically active substance and a droplet ejection device, for example a bubble jet or piezoelectric device, which is controlled to issue a predetermined number of discrete droplets of the substance from ejection orifices upon actuation. Device may be actuated by a pressure transducer responsive to inhalation and issue the droplets into an airstream (A) which enters at slot and is then inhaled via mouthpiece. In other embodiments (Fig. 5) the dispenser is finger actuated and directed by hand for topical application. The number and/or frequency of droplets issued is programmatically controlled by a control circuit whereby average and total dose of the substance are predetermined.

### DISCLOSURE OF INVENTION

According to a first aspect of the present invention, there is provided a flavor generation article characterized by comprising:
a casting having an air intake port for taking in air therein and a suction port through which a user inhales a flavor, and forming a gas flow path between said intake port and said suction port;
a material container for storing a liquid material which contains at least a flavor substance and having a discharge port for said material, said material container being mounted on said casing;
discharge driving means electrically operable for discharging said material from said container through said discharge port in the form of a liquid drop;
gasifying means disposed in said gas flow path to receive the liquid drop of said material discharged from said container by said discharge driving means and gasify said material by electrically heating the liquid drop;
a power supply for supplying electric energy to said discharge driving means and said gasifying means; and
control means responsive to suction applied to the suction part for selectively forming a state where said discharge driving means and said gasifying means operate in response to the suction by electric energy from said power supply.

According to a second aspect, there is a provide a flavor generation article in the first aspect, characterized by further comprising a sensor for detecting an inhaling operation of the user, wherein said control means controls, based on a signal from said sensor, said discharge driving means and said gasifying means so as to discharge said material from said container and to generate heat by said gasifying means.

According to the third aspect of the present invention, there is provided a flavor generation article in the second aspect, characterized in that the sensor comprises a pressure-sensitive sensor mounted on the casing around the suction port.

According to the fourth aspect of the present invention, there is provided a flavor generation article in the second or third aspect, characterized in that the control means controls the gasifying means based on the signal from the sensor so that the gasifying means generates heat.

According to the fifth aspect of the present invention, there is provided a flavor generation article in the fourth aspect, characterized in that the control means controls the gasifying means and the discharge driving means so as to preheat the gasifying means prior to discharge of the material.

According to the sixth aspect of the present invention, there is provided a flavor generation article in the first aspect, characterized in that the power supply is disposed in the casing.

According to the seventh aspect of the present invention, there is provided a flavor generation article in the sixth aspect, characterized in that the casing is constituted by first and second portions that are electrically connected to each other through a cable, the gas flow path, the container, the discharge driving means, and the gasifying means being disposed in the first portion, and the power supply being disposed in the second portion.

According to the eighth aspect of the present invention, there is provided a flavor generation article in the seventh aspect, characterized in that the first and second portions of the casing are detachably connected to each other through a connecting portion.

According to the ninth aspect of the present invention, there is provided a flavor generation article in the first aspect, characterized by further comprising an operation lever for manually operating the discharge driving means.

According to the 10th aspect of the present invention, there is provided a flavor generation article in any one of first to ninth aspects, characterized in that the gasifying means comprises a porous layer, and the liquid drop of the material is supplied onto the porous layer.

According to the 11th aspect of the present invention, there is provided a flavor generation article in any one of the first to 10th aspects, characterized in that the gasifying means is arranged to oppose the discharge port, and a throttle hole for directing air flowing from the air intake port toward a gap between the discharge port and the gasifying means is disposed in the gas flow path.

According to the 12th aspect of the present invention, there is provided a flavor generation article in any one of first to 11th aspects, characterized in that the casing is formed with an outer air inlet hole to supply an outer air into the gas flow path between the gasifying means and the suction port.

According to the 13th aspect of the present invention, there is provided a flavor generation article in any one of first to 12th aspects, characterized by further comprising a formed body of a solid material containing at least a flavor substance and disposed in the gas flow path so as to be located between the gasifying means and the suction port.

According to the 14th aspect of the present invention, there is provided a flavor generation article in the 13th aspect, characterized by further comprising heating means for heating the formed body.

According to the present invention, a flavor generation article can be provided in which waste of a flavor material does not occur easily and the timing of one puffing operation of the user and that of generation of a flavor can be matched easily. In particular, when the discharge driving means is controlled based on a signal from a sensor that detects the inhaling operation of the user, not only waste of the material is eliminated, but also a stable flavor can constantly be provided. When the casing is divided into a portion incorporating a power supply and a portion to be held by the mouth such that the two portions are detachable from each other, the flavor generation article can be used more conveniently.

### Brief Description of Drawings

FIG. 1 is a schematic view showing a flavor generation article according to an embodiment of the present invention;
FIG. 2 is a plan view showing the discharge head of the flavor generation article shown in FIG. 1;
FIG. 3 is an enlarged schematic view showing the discharge head and discharge drive portion taken along the line III - III of FIG. 2;
FIG. 4 is a diagram showing the control system of the flavor generation article shown in FIG. 1;
FIG. 5 is a view showing the state of use of the flavor generation article shown in FIG. 1;
FIG. 6 is a graph showing an example of operation timing of energization of the ceramic heater and that of actuation of the discharge drive portion, of the flavor generation article shown in FIG. 1;
FIG. 7 is a graph showing another example of operation timing of energization of the ceramic heater and that of actuation of the discharge drive portion, of the flavor generation article shown in FIG. 1;
FIG. 8 is a schematic view showing a flavor generation article according to another embodiment of the present invention;
FIG. 9 is a schematic view showing a flavor generation article according to still another embodiment of the present invention;
FIG. 10 is a schematic view showing a flavor generation article according to still another embodiment of the present invention;
FIG. 11 is a schematic view showing a flavor generation article according to still another embodiment of the present invention;
FIG. 12 is a schematic view showing a flavor generation article according to still another embodiment of the present invention;
FIG. 13 is a schematic view showing a flavor generation article according to still another embodiment of the present invention;
FIG. 14 is a schematic view showing a flavor generation article according to still another embodiment of the present invention;
FIG. 15 is a schematic view showing a flavor generation article according to still another embodiment of the present invention; and
FIG. 16 is a schematic view showing a flavor generation article according to still another embodiment of the present invention.

### Best Mode of Carrying Out the Invention

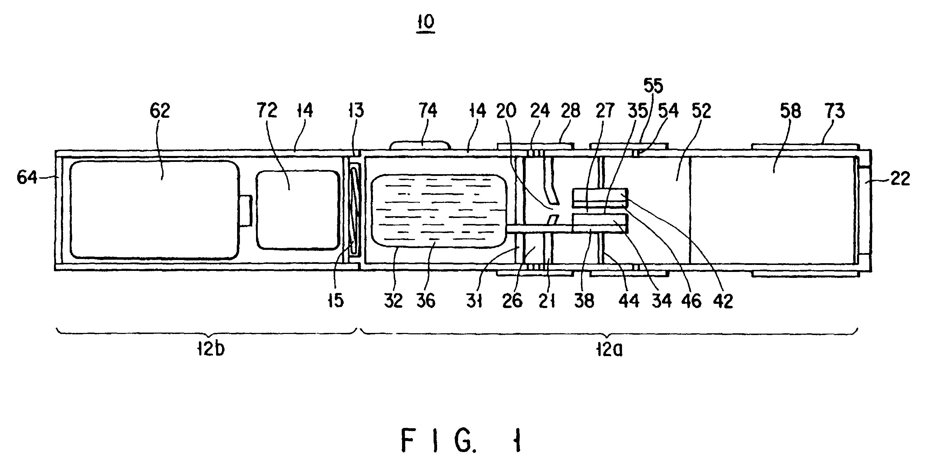

FIG. 1 is a schematic view showing a flavor generation article according to an embodiment of the present invention.

A flavor generation article 10 has a cylindrical casing 12 having such an outer diameter that the user can hold the casing 12 in his mouth. The casing 12 comprises a first portion 12a to be held by the user's mouth, and a second portion 12b for incorporating a power supply and the like. The two portions 12a and 12b are detachably connected to each other through a connecting portion 13 formed on a casing main body 14. The two portions 12a and 12b are electrically connected to each other through a cable 15 stored in a space formed in the casing main body 14 to correspond to the connecting portion 13. As the connecting portion 13, a known structure, e.g., a screw or a fitting pair, can be employed. The main body 14 of the casing 12 is made of a material, e.g., a plastic, metallic, ceramic, or wooden material.

A suction port 22 through which the user inhales the flavor is formed in the end portion of the first portion 12a of the casing 12. In contrast to this, a plurality of air intake ports 24 for taking in air into the casing 12 are formed in the intermediate portion of the first portion 12a. A gas flow path 26 is defined in the casing 12 between the air intake ports 24 and the suction port 22. The air intake ports 24 can be formed to have an open area corresponding to a predetermined air intake amount. As shown in FIG. 1, an adjusting ring 28 having a plurality of openings can be disposed on the casing 12 around the air intake ports 24. In this case, the amount of air flowing into the casing 12 can be adjusted by adjusting the position of the adjusting ring 28 with respect to the air intake ports 24.

A throttle plate 21 having a throttle hole 20 at its center is disposed in the casing 12 to be located in the gas flow path 26. The throttle hole 20 serves to regulate air from the air intake ports 24 to flow along the surface of a ceramic heater 42 (to be described later).

A material container 32 for storing a liquid material 36 for generating a flavor or the like to be inhaled by the user is detachably fixed in a space which is deep in the first portion 12a of the case and partitioned from the gas flow path 26 by a wall 31. The material container 32 stores the liquid material 36 in an amount corresponding to the discharge amount of a plurality of puffing operations of the user. The material container 32 can be mounted on the outer side of the casing main body 14. In this case, the head portion of the material container 32 may be inserted in the casing main body 14, or only discharge ports 35 (to be described later) may be inserted in the casing main body 14.

The liquid material 36 contains at least a flavor substance. For example, if the liquid material 36 is an article used for enjoying only the flavor, e.g., menthol or caffeine, it can be a material that generates only the flavor. Also, in order to add smoke to the flavor, the liquid material 36 can contain a material which generates aerosol when heated. As the material that generates aerosol, alcohols, saccharide, or water, or a mixture of at least two of these components can be used. The alcohols used in this case are, e.g., glycerin or propylene glycol, or their mixture.

More specifically, the liquid material 36 can contain an extracted material and/or the constituent components of various types of natural materials in accordance with the application purpose. For example, if this article is used as a simulated smoking article, a tobacco component, e.g., a tobacco extracted component or a tobacco smoke condensate component, may be contained in the liquid material 36.

The material container 32 is formed with a discharge head 34 having the plurality of discharge ports 35 for discharging the liquid material 36 in a transverse direction of the casing 12. The discharge head 34 is arranged to be located closer to the suction port 22 than the throttle hole 20. A discharge drive portion 38 is disposed adjacent to the discharge ports 35 to discharge the liquid material 36 from the material container 32 through the discharge ports 35. The discharge head 34 and the discharge drive portion 38 comprise a liquid discharge mechanism (having the same principle as that of the method shown in Jpn. Pat. Appln. KOKOKU Publication No. 53-45698 and U.S.P. No. 3,596,275) utilizing a piezoelectric element.

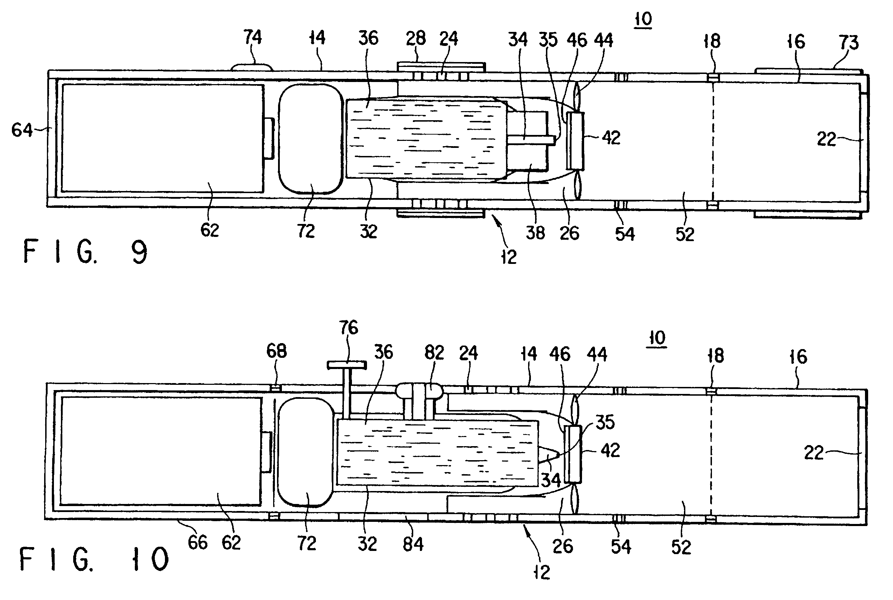

For example, as shown in FIG. 2, 10 discharge ports 35 are arranged for two rows, leading to a total of 20 discharge ports 35 in a region with a width W of about 2 mm and a length L of about 5 mm of the upper surface of the discharge head 34. The center of arrangement of the discharge ports 35 almost coincides with the center of the ceramic heater 42 (to be described later).

FIG. 3 is an enlarged schematic view showing the discharge head 34 and discharge drive portion 38 taken along the line III - III of FIG. 2. More specifically, FIG. 3 shows a section corresponding to one row of the discharge ports 35. A section corresponding to the other row of the discharge ports 35 and the section shown in FIG. 3 are horizontally symmetrical.

As shown in FIG. 3, a frame 134 constituted by a plurality of components is stacked on a wiring board 132 to form recessed portions and holes to be filled with the liquid material 36. The recessed portions formed by the frame 134, excluding the plurality of discharge ports 35, are covered with a film 136. A liquid reservoir 146 is formed under the discharge ports 35 to temporarily store the liquid material 36. The bottom plate of the liquid reservoir 146 is constituted by an electrode 138 that serves as a vibration plate.

The liquid material 36 from the material container 32 is supplied first through a narrow flow path 142, and flows from a plurality of suction holes 144, having a smaller diameter than that of the discharge ports 35, to reach the liquid reservoir 146. Under the control of a control circuit 72, when the electrode 138 is operated to vibrate, the liquid material 36 is selectively discharged through the discharge ports 35 having a low resistance against the flow. The discharged liquid material 36 is supplied onto the ceramic heater 42 as a liquid drop LD.

Other than this, as the discharge mechanism of the liquid material 36, a known printer ink discharge mechanism can be modified and employed, e.g., a method disclosed in Jpn. Pat. Appln. KOKOKU Publication No. 61-59911 and the like wherein the process liquid is injected by bubbles generated by heating it, or a method disclosed in U.S.P. No. 3,060,429 and the like wherein the particles of the process liquid are electrified to perform electric field control. Alternatively, a discharge mechanism in which a liquid material 36 is a pressurized liquid and is controlled by opening/closing a valve disposed in a discharge ports 35 may be employed.

The ceramic heater 42 is disposed in the gas flow path 26 to oppose the discharge ports 35. The ceramic heater 42 is fixed on the inner surface of the casing main body 14 through a support member 44. A gap 27 between the discharge ports 35 of the discharge head 34 and the ceramic heater 42 is set such that air from the throttle hole 20 can flow through it. Accordingly, air from the air intake ports 24 is directed by the throttle hole 20 to the gap 27 between the discharge ports 35 and ceramic heater 42.

A material corresponding to one puffing operation, which is driven by the discharge drive portion 38 and emitted from the discharge ports 35 is supplied onto the ceramic heater 42 in the form of a liquid splash or liquid drop. The ceramic heater 42 is constituted by a ceramic plate and a coated resistance heater on the ceramic plate, and is accordingly an integral member of a catch pan for receiving the splash of the material and a heating means for heating the catch pan. However, the catch pan and the heating means can be disposed as separate components.

A liquid-absorbing porous layer 46 having a thickness of 0.01 mm to 2.0 mm, e.g., an activated carbon layer having a thickness of about 0.5 mm, is formed on a surface of the ceramic heater 42 that receives the liquid splash of the material, i.e., a surface of the ceramic heater 42 that serves as the catch pan. The porous layer 46 not only protects the surface of the ceramic heater 42 but also relaxes heat conduction from the ceramic heater 42, thereby stabilizing gasification of the splash of the material. The porous layer 46 can be formed of an organic compound, e.g., natural cellulose, a cellulose derivative, or an aramid resin, or an inorganic compound, e.g., carbon (including activated carbon), alumina, or silicon carbide. The porous layer 46 can have an arbitrary shape. For example, the compound mentioned above may be formed as a formed body in advance, e.g., a film, a sheet, a plate, fabric, or unwoven fabric, and be used as the porous layer 46. Alternatively, the porous layer 46 may be formed by directly applying the powder of the component mentioned above on the ceramic heater 42.

A cooling chamber 52 is formed between the ceramic heater 42 and the suction port 22 to constitute part of the gas flow path 26. Outer air inlet holes 54 are formed in the side wall of the casing main body 14 defining the cooling chamber. The gas heated by the ceramic heater 42 and containing a flavor is mixed with the outer air and cooled in the cooling chamber 52, and reaches the suction port 22. The outer air inlet holes 54 can be formed to have an open area corresponding to a predetermined air inlet amount. As shown in FIG. 1, an adjusting ring 55 having a plurality of openings can be disposed on the casing 12 around the outer air inlet holes 54. In this case, the amount of outer air flowing into the cooling chamber 52 can be adjusted by adjusting the position of the adjusting ring 55 with respect to the outer air inlet holes 54.

A filter 58 is disposed in the gas flow path 26 between the cooling chamber 52 and suction port 22 to cover the suction port 22. When the filter 58 is disposed, the pressure loss can be adjusted so that the flavor component can be inhaled with an appropriate pressure. The filter 58 can be made of a normal tobacco filter material made of cellulose acetate, pulp, or the like.

A power supply 62 is detachably fixed in the second portion 12b of the casing 12. The power supply 62 is used to supply electric energy to the discharge drive portion 38, the ceramic heater 42, and the control circuit 72 (to be described later). The power supply 62 can be mounted in and removed from the casing main body 14 by opening/closing a cap 64 that closes the rear opening of the casing main body 14. The power supply 62 is preferably a DC power supply, e.g., a commercially available dry cell or rechargeable cell. However, the power supply 62 can be an AC power supply. The power supply 62 can be mounted on the outer side of the casing main body 14, or can be provided separately and connected to the casing main body 14 with a wire.

The control circuit 72 for controlling the driving operation of the discharge drive portion 38 and the ceramic heater 42 is arranged between the power supply 62 and material container 32. As shown in FIG. 4, the control circuit 72 has a signal processing circuit 72a, a drive circuit 72b, and a power circuit 72c. The signal processing circuit 72a is connected to a sensor 73 for detecting the inhaling operation of the user and a manual ON/OFF switch 74. The drive circuit 72b is connected to the discharge drive portion 38 and the ceramic heater 42. The power circuit 72c is connected to the power supply 62.

The sensor 73 for detecting the inhaling operation of the user is disposed around the casing main body 14 to be adjacent to the suction port 22. The sensor 73 has the same principle as that of a general strain type pressure-sensitive sensor for detecting a change in resistance or capacitance, a piezoelectric electromotive force, or the like, and generates an electrical signal upon detection of a pressure with which the user holds the casing 12 in his mouth. Alternatively, as the sensor 73, a swing vane type sensor (to be described later), a contact type sensor, a lip sensor disclosed in Jpn. Pat. Appln. KOKAI Publication No. 5-212100, or the like can be used.

Upon reception of a signal from the manual ON/OFF switch 74, or based on a signal from the sensor 73, the control circuit 72 starts the discharge drive portion 38 and the ceramic heater 42 at a timing to match the inhaling operation of the user, so that the liquid material is discharged and gasified. For example, signal processing of the control circuit 72 and the way of control of the control circuit 72 can be known as analog control or two-position control, or their combination.

The manual ON/OFF switch 74 is disposed on the side surface of the first portion 12a of the casing 12. When this article is not in use, the switch 74 may be manually switched to the OFF state, thereby forcibly stopping the discharge drive portion 38 and the heater 42. The manual switch 74 has the same mechanism as that of a general compact push switch, e.g., a micro limit switch having an electric contact.

How the flavor generation article 10 shown in FIG. 1 is operated will be described.

When the user performs simulated smoking or inhales the flavor by using the flavor generation article 10 shown in FIG. 1, first, the user turns on the manual switch 74, holds the first portion 12a of the casing 12 with his mouth, and performs an inhaling operation through the suction port 22. By this operation, the sensor 73 outputs an inhaling operation signal to the control circuit 72. Accordingly energization of the ceramic heater 42 is started under the control of the control circuit 72. Simultaneously, or with a lapse of a predetermined period after the start of energization, the discharge drive portion 38 is actuated.

The liquid material 36 is then discharged from the discharge ports 35 and gasified as it is heated by the ceramic heater 42. As the user performs an inhaling operation, the gasified material is mixed with main suction air which has been taken in from the air intake ports 24, passed through the throttle hole 20, and guided to a portion between the discharge ports 35 and ceramic heater 42, and is guided to the suction port 22.

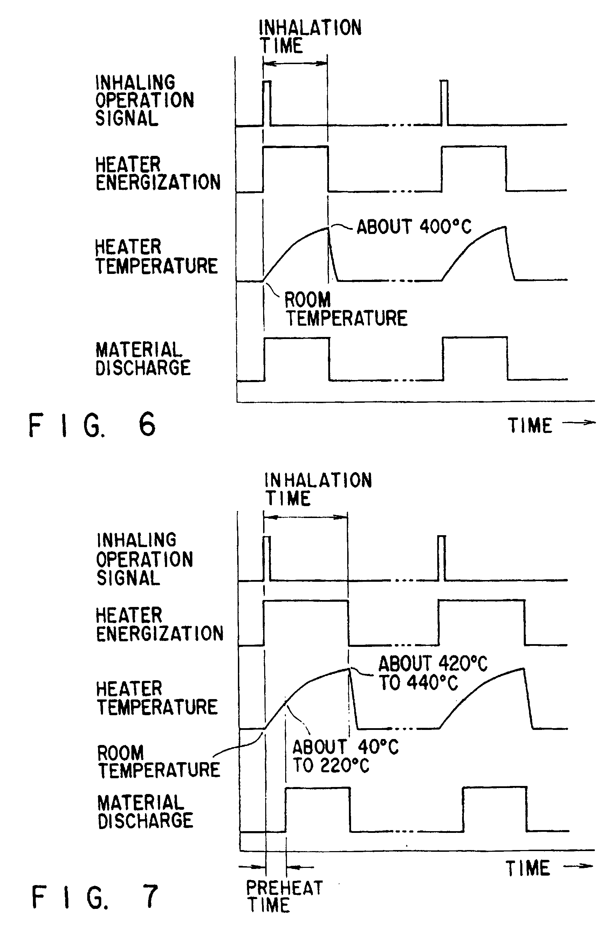

Energization of the ceramic heater 42 and actuation of the discharge drive portion 38 are performed, e.g., at the operation timings shown in FIG. 6 or 7. FIG. 6 shows a case wherein, in response to a signal from the sensor 73, the ceramic heater 42 is energized and heated and the liquid material 36 is discharged simultaneously. FIG. 7 shows a case wherein, in response to a signal from the sensor 73, the ceramic heater 42 is energized and preheated in advance, and with a lapse of a predetermined period of time, i.e., when the heater temperature has increased to a certain degree, the liquid material 36 is discharged.

If necessary, the amount of main suction air taken in from the air intake ports 24 and the amount of inlet air supplied from the outer air inlet holes 54 can be changed by adjusting the adjusting rings 28 and 60 during inhalation. Then, the taste of air containing the flavor and reaching the suction port 22 can be changed, so that the user can perform simulated smoking or inhalation of the flavor in accordance with the taste of his inhalation feeling.

As described above, the casing 12 has a structure in which the first portion 12a storing the liquid material 36, the discharge head 34, the ceramic heater 42, and the like, and the second portion 12b storing the control circuit 72, the power supply 62, and the like are detachably connected to each other through the connecting portion 13. The first and second portions 12a and 12b are electrically connected to each other through the cable 15. Therefore, this flavor generation article 10 may be used with its first and second portions 12a and 12b being integrally connected to each other through the connecting portion 13, or may be used with its first and second portions 12a and 12b being separated from each other, as shown in FIG. 5. In the state shown in FIG. 5, since the first and second portions 12a and 12b can be separated within a range allowed by the cable 15, for example, the user can place the second portion 12b in his pocket and hold only the first portion 12a in his mouth. Alternatively, the second portion 12b separated from the first portion 12a may be connected to an existing power supply, i.e., may be installed.

Several experiments using the flavor generation article 10 shown in FIG. 1 will be described.

First, as the flavor substance, some natural peppermint oil was used, and as the aerosol generation material to add smoke to the flavor, glycerin was used. Water was added to the natural peppermint oil and glycerin, thereby preparing a plurality of liquid materials 36 in which the water to glycerin concentration ratio changed in a range of about 2 : 98 to about 90 : 10. Aerosol containing a flavor substance obtained by heating each liquid material 36 was inhaled, by using the flavor generation article shown in FIG. 1, with a standard smoking condition of one cycle for about one minute in which 35 cc to 50 cc of aerosol were inhaled in one inhaling operation for about 2 seconds with an interval of about 58 seconds.

As a result, when a liquid material having a water to glycerin concentration ratio of 50 : 50 and prepared by adding some natural peppermint oil was employed as the liquid material 36, sufficiently high discharge stability was ensured, and physical satisfaction and requirement for a visually observed smoke amount upon inhalation were achieved to a certain degree. Therefore, in the following experiments, this liquid material was used as the liquid material 36. In the following experiments as well, inhalation was performed with the standard smoking condition of one cycle for about one minute in which 35 cc to 50 cc of aerosol were inhaled in one inhaling operation for about 2 seconds with an interval of about 58 seconds, and a discharge speed of about 2.5 mg/second was employed.

With this condition, the operation timings shown in FIGS. 6 and 7 were compared. First, at the timing shown in FIG. 6, the heater was heated from room temperature to about 400°C within 2 seconds. In this case, the liquid material 36 accumulated on the heater surface while the heater increased to the temperature that enabled gasification was gasified at once, and was condensed near the discharge ports 35 because of rapid expansion or flied in the form of a liquid drop because of bumping, thus decreasing the yield. Subsequently, the heater was preheated to about 140°C to 220°C during the preheat time at the timing shown in FIG. 7, and was thereafter heated to 420°C to 440°C within 2 seconds. In this case, the liquid material 36 was effectively gasified in an interlocked manner with discharge.

The inhalation time of the user should correspond to a time period between the start and end of energization of the heater and discharge in FIG. 6, and should correspond to a time period between the start and end of energization of the heater, including the preheat time, in FIG. 7. Accordingly, the preheat time is preferably set within a range of about 0.1 second to 1 second in the standard smoking time, so that the user will not feel discomfort during inhalation, and it is required that the preheat time is not so high.

For example, when the heater was preheated to about 400°C with a preheat time of 2 seconds, the material discharged after that was rapidly gasified and expanded. Then, the proportion of the material that was condensed near the discharge ports 35 increased, inversely decreasing the yield. Also, since the inhalation operation was allowed with the lapse of 2 seconds after the user held the sensor 73 of the suction port 22 in his mouth, a time lag occurred to make the user feel discomfort. In this experiment, with the operation timings shown in FIG. 7, preheat from room temperature to 140°C spent a preheat time of about 0.5 second, and preheat to 220°C spent a preheat time of about 1 second.

When the surface of the ceramic heater 42 had no porous layer 46 but was flat, a phenomenon in which the liquid material 36 was not easily caught by the heater surface but was bounded was observed. In this case, at either timings shown in FIG. 6 or 7, the yield tended to decrease.

Regarding the main inhalation air which flowed through the throttle hole 20 and passed through the gap 27, the higher the flow velocity to a certain degree, the better the gasification efficiency of the liquid material. Concerning this, under the standard smoking condition of 35 cc to 50 cc per inhalation for 2 seconds, a desired result was obtained when the position of the throttle hole 20 was within about 30 mm from the center of the gap 27 and the velocity of air passing through the throttle hole 20 was equal to or higher than about 6 m/second. This corresponds to the sectional area of the opening of the throttle hole 20 of about 3 mm² or less. However, it is nonsense to decrease the sectional area of the opening (to increase the flow velocity) to such a degree that it becomes impossible for the user to perform inhalation with his mouth. Considering the above respects, the lower limit of the sectional area of the opening of the throttle hole 20 is supposed to be preferably about 0.6 mm².

The size of the gap 27, i.e., the vertical distance between the discharge ports 35 and ceramic heater 42 also influenced the gasification efficiency of the liquid material 36. In order to suppress a decrease in yield caused by condensation of the gas near the discharge ports 35, the ceramic heater 42 and discharge ports 35 must oppose each other through a distance equal to or larger than about 2 mm.

Several flavor generation articles according to other embodiments of the present invention will be described. In the drawings indicating these embodiments, portions that are common to the preceding drawings are denoted by the same reference numerals, and a detailed description thereof will be omitted.

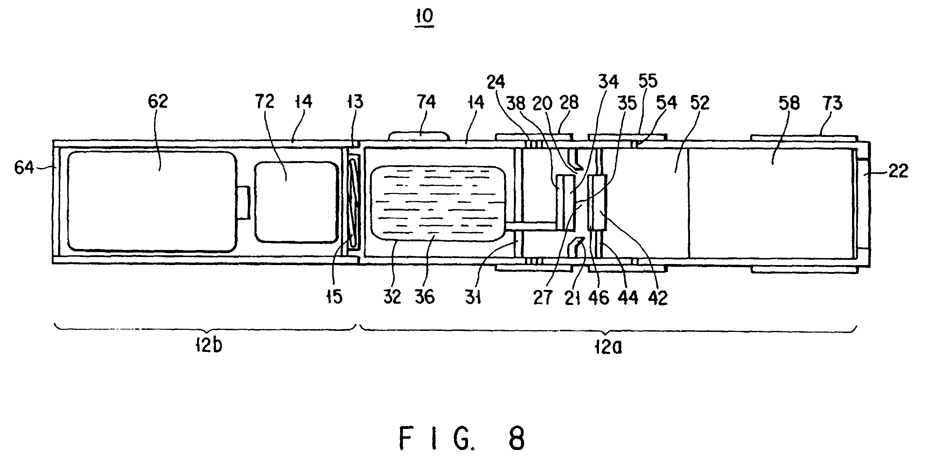

FIG. 8 is a schematic view showing a flavor generation article according to another embodiment of the present invention.

The flavor generation article of this embodiment is similar to the flavor generation article shown in FIG. 1, but the orientation of discharge ports 35 of a discharge head 34 is different from that of the structure shown in FIG. 1 by 90° , so that the discharge ports 35 may be directed to a suction port 22. Accordingly, a ceramic heater 42 opposing the discharge ports 35 is set such that its direction is different from that of the structure shown in FIG. 1 by 90° . Since the discharge head 34 is arranged in a throttle hole 20, the substantial opening of the throttle hole 20 that serves as a gas flow path 26 is regulated by the size of both the throttle hole 20 and discharge head 34.

FIG. 9 is a schematic view showing a flavor generation article according to still another embodiment of the present invention.

The characteristic feature of the flavor generation article of this embodiment resides in that, first, a casing 12 cannot be separated into first and second portions 12a and 12b (see FIG. 1), and a liquid material 36, a discharge head 34, a ceramic heater 42, a power supply 62, a control unit 72, and the like are incorporated in one casing main body 14. However, a mouthpiece 16 is detachably mounted on the casing main body 14 through a connecting portion 18, and a suction port 22 is formed in the mouthpiece 16. The mouthpiece 16 is made of a material, e.g., a plastic or wood. As the connecting portion 18, a known structure, e.g., a screw or a fitting pair can be employed. In place of the mouthpiece 16, a filter may be inserted in the casing main body 14 and served for use.

The discharge head 34 provided to a material container 32 has one discharge port 35 which is oriented to discharge the liquid material 36 toward the suction port 22. Accordingly, the ceramic heater 42 opposing the discharge port 35 is oriented in the same direction as that of the structure shown in FIG. 8. No throttle plate 21 (see FIG. 1) is disposed in a gas flow path 26. Air that has flowed into the article flows on the ceramic heater 42 because it is regulated by a support member 44 supporting the ceramic heater 42.

FIG. 10 is a schematic view showing a flavor generation article according to still another embodiment of the present invention.

The flavor generation article of this embodiment is similar to the flavor generation article shown in FIG. 9 but is largely different from it in that its material container 32 is manually operated to discharge. For this reason, the material container 32 is connected to an operation lever 76 projecting outside a casing main body 14. When the lever 76 is depressed, a material corresponding to one puffing operation is emitted from a discharge port 35, and is supplied onto a ceramic heater 42 in the form of a liquid splash or liquid drop. A control circuit 72 receives a signal indicating a depressing operation of the lever 76, and supplies power to the ceramic heater 42 based on this signal to heat it, thereby gasifying the material splash. In fine, the lever 76 serves as both the discharge drive portion 38 for the flavor generation article and the sensor 73 for detecting the inhaling operation of the user that are shown in FIG. 1.

In the flavor generation article shown in FIG. 10, the material container 32 is also connected to an injection port 82 for replenishing the material container 32 with a liquid material 36. The end portion of the injection port 82 is exposed outside the casing main body 14, and the liquid material can be injected and replenished to the material container 32 through this end portion. As described above, the material container 32 has a capacity sufficient for storing the liquid material 36 in an amount corresponding to the total discharge amount of a plurality of puffing operations of the user. However, if the material can be replenished, the material container 32 need not be exchanged, but this flavor generation article can be used further continuously.

In order to observe the remaining amount in the material container 32, a transparent inspection window 84 is formed in the side wall of the casing main body 14 to correspond to the material container 32. Accordingly, in this case, the material container 32 itself is also a transparent or translucent container. When the remaining amount of the liquid material 36 in the material container 32 is monitored through the inspection window 84, the user can know the timing at which the container should be replenished with the material.

In place of the arrangement shown in FIG. 10, a combination of an electric remaining amount detection means and an electric display means can be used to monitor the remaining amount in the material container 32. An example of the electric remaining amount detection means includes a means for detecting a change in conductivity of the material container 32, and an example of the electric display means includes a means for using a light-emitting diode disposed on the outer surface of the casing main body 14. As the mechanism for monitoring the remaining amount in the material container 32, a method that optically detects the remaining amount by using a prism may also be employed.

In the flavor generation article shown in FIG. 10, furthermore, a power supply 62 is stored in a power supply holder 66 which is detachably mounted on the casing main body 14 through a connecting portion 68. As the connecting portion 68, a known structure, e.g., a screw or a fitting pair, can be employed. When the power supply holder 66 having a length corresponding to the size of the power supply 62 is used, exchange of the power supply 62 is facilitated, and repair and exchange of members in the casing main body 14 are also facilitated.

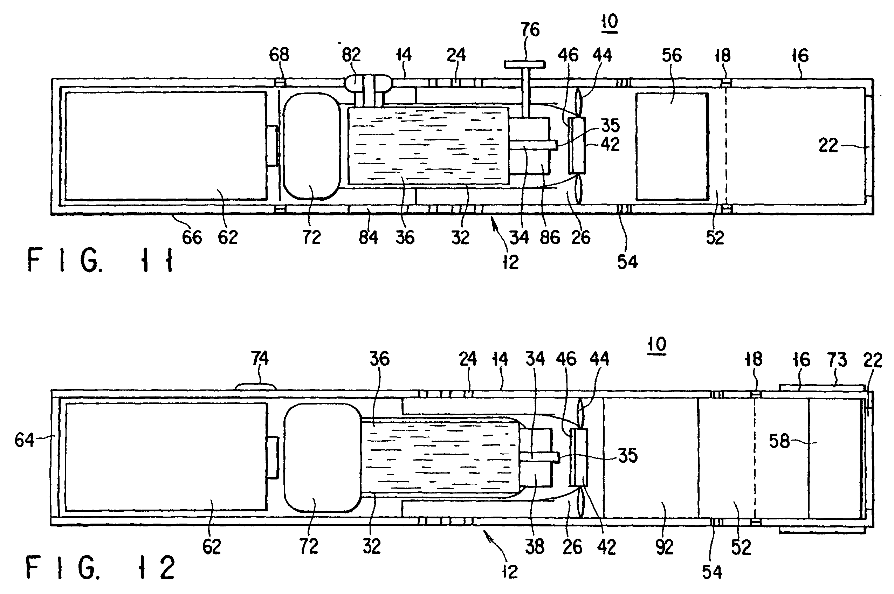

FIG. 11 is a schematic view showing a flavor generation article according to still another embodiment of the present invention.

The flavor generation article of this embodiment is similar to the flavor generation article shown in FIG. 10 but is different from it in that a discharge operation lever 76 is connected to an atomizer 86 provided to a discharge port 35. The atomizer 86 can supply a material corresponding to one puffing operation onto a ceramic heater 42 in the form of a liquid splash or liquid drop.

In the flavor generation article shown in FIG. 11, a filler 56 is disposed in a cooling chamber 52. When the filler 56 is disposed, the cooling effect of the gasified flavor component can be promoted, and the pressure loss can be adjusted so that the flavor component can be inhaled with an appropriate pressure. As the filler 56, for example, a fiber formed body made of cellulose acetate or pulp, or a particulate matter, e.g., glass or aluminum particles, can be used.

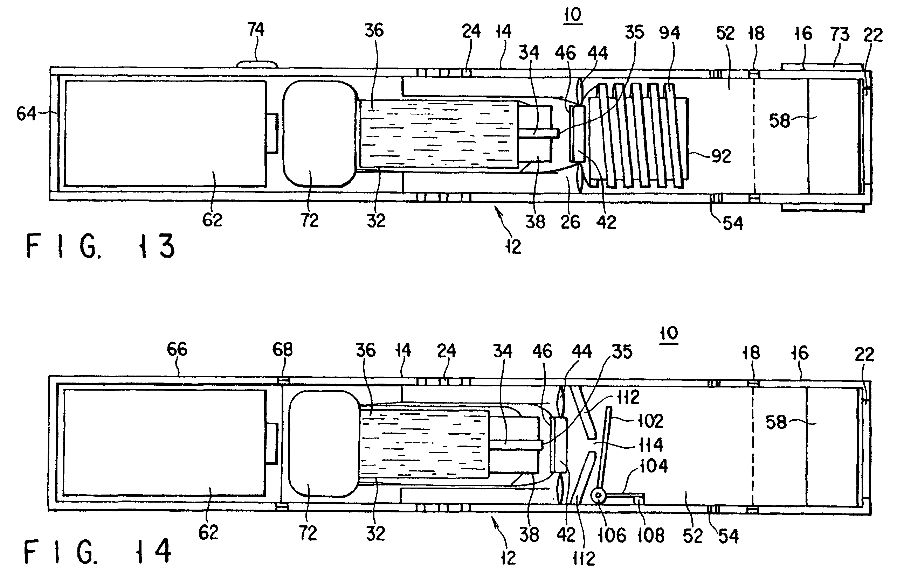

FIG. 12 is a schematic view showing a flavor generation article according to still another embodiment of the present invention.

The characteristic feature of the flavor generation article of this embodiment resides in that a formed body 92 of a solid material that generates a flavor or the like to be inhaled by the user is detachably disposed in a gas flow path 26 between a ceramic heater 42 and a cooling chamber 52.

The formed body 92 of the solid material can contain an extracted material and/or the constituent components of various types of natural materials in accordance with the application purpose. As the flavor material to be contained by the formed body 92, for example, menthol, caffeine, or a tobacco component, e.g., a tobacco extracted component or a tobacco smoke condensate component can be employed.

If the formed body 92 of the solid material has such a size that no gap is formed between it and the inner surface of a casing main body 14, a formed body 92 having good air permeability is used as the formed body 92. In this case, the gas flow path 26 between air intake ports 24 and a suction port 22 is formed to extend through the formed body 92. On the other hand, if the size of the formed body 92 is set such that a gap is formed between the formed body 92 and the inner surface of the casing main body 14, a formed body 92 having poor or no air permeability can be used. In this case, the gas flow path 26 between the air intake ports 24 and suction port 22 is formed to extend through the gap between the formed body 92 and the inner surface of the casing main body 14.

FIG. 13 is a schematic view showing a flavor generation article according to still another embodiment of the present invention.

The flavor generation article of this embodiment is different from the flavor generation article shown in FIG. 12 in that a coil heater 94 for heating a formed body 92 is disposed around the formed body 92. The heater for heating the formed body 92 may be arranged in a hole formed in the formed body 92.

The coil heater 94, together with a ceramic heater 42, can be controlled by a control circuit 72 so that power is supplied to them in accordance with the inhaling operation of the user. When the formed body 92 has a large heat capacity, however, even if power is supplied to the coil heater 94 in accordance with the start of the inhaling operation of the user, generation of the flavor may be retarded considerably. In such a case, the coil heater 94 may be kept heated when this article is in use, i.e., while a switch 74 is set in the ON state.

The formed body 92 has such a size that a sufficiently large gap is formed between it and the inner surface of a casing main body 14. Accordingly, the major portion of a gas flow path 26 between air intake ports 24 and a suction port 22 extends through this gap.

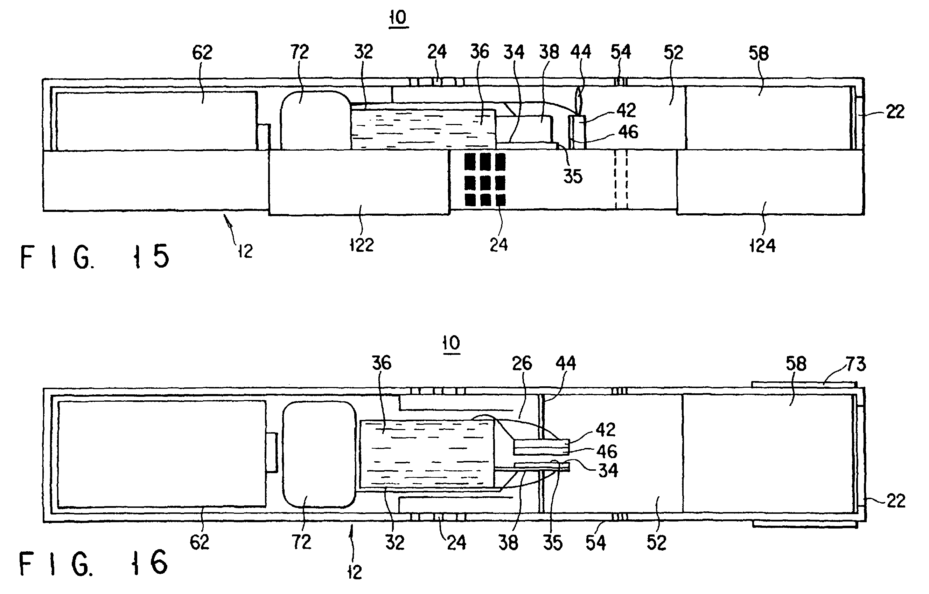

FIG. 14 is a schematic view showing a flavor generation article according to still another embodiment of the present invention.

The characteristic feature of the flavor generation article according to this embodiment resides in that a swing vane type sensor is used to detect the inhaling operation of the user. More specifically, a swing vane 102 is disposed in a gas flow path 26 between a ceramic heater 42 and a cooling chamber 52. An orifice 112 having an opening 114 opposing the vane 102 is disposed in the gas flow path 26 between the ceramic heater 42 and the vane 102. The vane 102 is integrally connected to a conductive lever 104 which serves as the switch lever of the sensor circuit. An electric contact 108 of the sensor circuit is disposed on the inner surface of a casing main body 14 to oppose the conductive lever 104.

The vane 102 and lever 104 are integrally, swingably, and axially supported on a support 106 on the inner surface of the casing main body 14, and is biased counterclockwise in FIG. 14 by a spring incorporated in the support 106. Accordingly, in an ordinary state, the vane 102 abuts against the orifice 112, and the lever 104 and contact 108 are not in contact with each other. However, when the user starts an inhaling operation, the gas flow, the flow velocity of which is increased by the orifice 112, pivots the vane 102 clockwise in FIG. 14, so that the lever 104 and contact 108 come into contact with each other. The inhaling operation signal of the user which is detected in this manner by the swing vane type sensor is transmitted to a control circuit 72. Based on this detection signal, a discharge drive portion 38 and the ceramic heater 42 can be controlled.

FIG. 15 is a schematic view showing a flavor generation article according to still another embodiment of the present invention.

The characteristic feature of the flavor generation article according to this embodiment resides in that a contact sensor is used in order to detect the inhaling operation of the user. More specifically, electric contacts 122 and 124 each made of an annular conductive plate are disposed at the center and the suction port-side end portion, respectively, of the outer surface of a casing 12. The electric contacts 122 and 124 constitute the switch of a sensor circuit. When the electric contacts 122 and 124 are connected to each other through a conductor, the sensor generates a detection signal. This state occurs when, e.g., two conditions that the user holds the electric contact 122 at the center with his hand and holds the suction port-side electric contact 124 in his mouth are satisfied simultaneously. The inhaling operation signal of the user which is detected by the contact sensor in this manner is transmitted to a control circuit 72. A discharge drive portion 38 and a ceramic heater 42 can be controlled based on this detection signal.

FIG. 16 is a schematic view showing a flavor generation article according to still another embodiment of the present invention.

The flavor generation article of this embodiment has discharge ports 35 of a discharge head 34 that are oriented in the same direction as that of the flavor generation article shown in FIG. 1, and a ceramic heater 42 opposing the discharge ports 35. However, a casing 12 cannot be separated into first and second portions 12a and 12b (see FIG. 1), and a liquid material 36, the discharge head 34, the ceramic heater 42, a power supply 62, a control unit 72, and the like are incorporated in one casing main body 14.

Although no throttle plate 21 (see FIG. 1) is disposed in a gas flow path 26, a support member 44 of the ceramic heater 42 is formed to have a slit only at its central portion corresponding to the discharge head 34. Accordingly, air flowing through air intake ports 26 entirely passes through the gap between the discharge port 35 and the ceramic heater 42.

The characteristic features of the respective portions of the present invention have been described divisionally by way of several embodiments in order to facilitate understanding of the present invention. These characteristic features can be appropriately combined in accordance with the object. More specifically, the present invention can be practiced in various embodiments other than those shown in the drawings within the scope of the invention, as defined by the appended claims.

## Claims

1. A flavor generation article **characterized by** comprising:
a casting (12) having an air intake port (24) for taking in air therein and a suction port (22) through which a user inhales a flavor, and forming a gas flow path (26) between said intake port and said suction port;
a material container (32) for storing a liquid material which contains at least a flavor substance and having a discharge port (35) for said material, said material container being mounted on said casing;
discharge driving means (38) electrically operable for discharging said material from said container through said discharge port in the form of a liquid drop;
gasifying means (42) disposed in said gas flow path to receive the liquid drop of said material discharged from said container by said discharge driving means and gasify said material by electrically heating the liquid drop;
a power supply (62) for supplying electric energy to said discharge driving means and said gasifying means; and
control means (72) responsive to suction applied to the suction part (22) for selectively forming a state where said discharge driving means and said gasifying means operate in response to the suction by electric energy from said power supply.

2. A flavor generation article according to claim 1, **characterized by** further comprising a sensor (73) for detecting an inhaling operation of the user, wherein said control means (72) controls, based on a signal from said sensor, said discharge driving means (38) and said gasifying means (42) so as to discharge said material from said container (32) and to generate heat by said gasifying means.

3. A flavor generation article according to claim 2, **characterized in that** said sensor (73) comprises a pressure-sensitive sensor mounted on said casing (12) around said suction port (22).

4. A flavor generation article according to claim 2 or 3, **characterized in that** said control means (72) controls said discharge driving means (38) and said gasifying means (42) based on the signal from said sensor (73) so that said discharge driving means and said gasifying means start operation at the same time.

5. A flavor generation article according to claim 2 or 3, **characterized in that** said control means (72) controls said gasifying means (42) and said discharge driving means (38) based on the signal from said sensor (73) so as to preheat said gasifying means prior to discharge of said material.

6. A flavor generation article according to any one of claims 1 to 5, **characterized in that** said control means (72) controls said gasifying means (42) and said discharge driving means (38) so that said discharge driving means and said gasifying means stop operation at the same time.

7. A flavor generation article according to claim 1, **characterized in that** said power supply (62) is disposed in said casing (12).

8. A flavor generation article according to claim 7, **characterized in that** said casing (12) is constituted by first and second portions (12a, 12b) that are electrically connected to each other through a cable (15), said gas flow path (26), said container (32), said discharge driving means (38), said gasifying means (42) being disposed in said first portion (12a), and said power supply (62) being disposed in said second portion (12b).

9. A flavor generation article according to claim 8, **characterized in that** said first and second portions (12a, 12b) of said casing (12) are detachably connected to each other through a connecting portion (13).

10. A flavor generation article according to any one of claims 1 to 9, **characterized in that** said gasifying means (42) comprises a porous layer (46), and the liquid drop of said material is supplied onto said porous layer.

11. A flavor generation article according to any one of claims 1 to 10, **characterized in that** said gasifying means (42) is arranged to oppose said discharge port (35), and a throttle hole (20) for directing air flowing form said air intake port (24) toward a gap between said discharge port and said gasifying means is disposed in said gas flow path (26).

12. A flavor generation article according to any one of claims 1 to 11, **characterized in that** said casing (12) is formed with an outer air inlet hole (54) in order to supply an outer air into said gas flow path (26) between said gasifying means (42) and said suction port (22).

13. A flavor generation article according to any one of claims 1 to 12, **characterized by** further comprising a formed body (92) of a solid material containing at least a flavor substance and disposed in said gas flow path (26) so as to be located between said gasifying means (42) and said suction port (22).

14. A flavor generation article according to claim 13, **characterized by** further comprising heating means (94) for heating said formed body (92).

## Patentansprüche

1. Aromaerzeugender Gegenstand **gekennzeichnet durch**:
ein Gehäuse (12) mit einer Lufteinlaßöffnung (24) zum Hereinlassen von Luft und einer Saugöffnung (22), **durch** welche ein Nutzer ein Aroma inhaliert, und die einen Gasströmungsweg (26) zwischen der Einlaßöffnung und der Saugöffnung bildet;
einen Materialbehälter (32) zum Lagern eines zumindest eine Aromasubstanz enthaltenden flüssigen Materials, welcher eine Abgabeöffnung (35) für das Material aufweist, wobei der Materialbehälter auf dem Gehäuse befestigt ist;
eine Abgabe-Antriebseinrichtung (38), welche elektrisch zur Abgabe des Materials aus dem Behälter **durch** die Abgabeöffnung in der Form eines flüssigen Tropfens betreibbar ist;
eine Vergasungseinrichtung (42), welche in dem Gasströmungsweg zum Aufnehmen des flüssigen Tropfens des Materials, welches von dem Behälter **durch** die Entlade-Antriebseinrichtung abgebbar ist, und zum Vergasen des Materials **durch** elektrisches Heizen des flüssigen Tropfens angeordnet ist;
eine Stromversorgung (62) zum Liefern von elektrischer Energie an die Entlade-Antriebseinrichtung und die Vergasungseinrichtung; und **durch**
eine Steuereinrichtung (72), welche auf ein an der Saugöffnung (22) auftretendes Saugen zum selektiven Bilden eines Zustands anspricht, in welchem die Entlade-Antriebseinrichtung und die Vergasungseinrichtung in Abhängigkeit vom Saugen **durch** elektrische Energie von der Stromversorgung betreibbar ist.

2. Aromaerzeugender Gegenstand nach Anspruch 1, **gekennzeichnet durch** einen Sensor (73) zum Erfassen eines Inhalationsvorgangs des Nutzers, wobei die Steuereinrichtung (72) basierend auf einem Signal von dem Sensor die Abgabe-Antriebseinrichtung (38) und die Vergasungseinrichtung (42) derart steuert, daß das Material von dem Behälter (32) abgegeben und Hitze **durch** die Vergasungseinrichtung erzeugt wird.

3. Aromaerzeugender Gegenstand nach Anspruch 2, **dadurch gekennzeichnet, daß** der Sensor (73) einen drucksensiblen Sensor aufweist, welcher auf dem Gehäuse (12) um die Saugöffnung (22) herum befestigt ist.

4. Aromaerzeugender Gegenstand nach Anspruch 2 oder 3, **dadurch gekennzeichnet, daß** die Steuereinrichtung (72) die Abgabe-Antriebseinrichtung (38) und die Vergasungseinrichtung (42) basierend auf dem Signal von dem Sensor (73) derart steuert, daß die Abgabe-Antriebseinrichtung und die Vergasungseinrichtung den Betrieb gleichzeitig starten.

5. Aromaerzeugender Gegenstand nach Anspruch 2 oder 3, **dadurch gekennzeichnet, daß** die Steuereinrichtung (72) die Vergasungseinrichtung (42) und die Abgabe-Antriebseinrichtung (38) basierend auf dem Signal von. dem Sensor (73) derart steuert, daß die Vergasungseinrichtung vor dem Entladen des Materials vorgeheizt wird.

6. Aromaerzeugender Gegenstand nach einem der Ansprüche 1 bis 5, **dadurch gekennzeichnet, daß** die Steuereinrichtung (72) die Vergasungseinrichtung (42) und die Abgabe-Antriebseinrichtung (38) derart steuert, daß die Abgabe-Antriebseinrichtung und die Vergasungseinrichtung den Betrieb gleichzeitig stoppen.

7. Aromaerzeugender Gegenstand nach Anspruch 1, **dadurch gekennzeichnet, daß** die Stromversorgung (62) in dem Gehäuse (12) angeordnet ist.

8. Aromaerzeugender Gegenstand nach Anspruch 7, **dadurch gekennzeichnet, daß** das Gehäuse (12) aus einem ersten und einem zweiten Abschnitt (12a, 12b) besteht, welche elektrisch durch ein Kabel (15) miteinander verbunden sind, wobei der Gasströmungsweg (26), der Behälter (32), die Abgabe-Antriebseinrichtung (38), die Vergasungseinrichtung (42) in dem ersten Abschnitt (12a) angeordnet sind, und die Stromversorgung (62) in dem zweiten Abschnitt (12b) angeordnet ist.

9. Aromaerzeugender Gegenstand nach Anspruch 8, **dadurch gekennzeichnet, daß** der erste und zweite Abschnitt (12a, 12b) des Gehäuses (12) abnehmbar miteinander durch einen Verbindungsabschnitt (13) verbunden sind.

10. Aromaerzeugender Gegenstand nach einem der Ansprüche 1 bis 9, **dadurch gekennzeichnet, daß** die Vergasungseinrichtung (42) eine poröse Schicht (46) aufweist, und der flüssige Tropfen des Materials auf die poröse Schicht lieferbar ist.

11. Aromaerzeugender Gegenstand nach einem der Ansprüche 1 bis 10, **dadurch gekennzeichnet, daß** die Vergasungseinrichtung (42) der Abgabeöffnung (35) gegenüberliegend angeordnet ist, und daß eine Drosselöffnung (20) zum Leiten von Luft von der Lufteinlaßöffnung (24) in Richtung eines Spalts zwischen der Abgabeöffnung und der Vergasungseinrichtung in dem Gasströmungsweg (26) angeordnet ist.

12. Aromaerzeugender Gegenstand nach einem der Ansprüche 1 bis 11, **dadurch gekennzeichnet, daß** das Gehäuse (12) mit einem äußeren Lufteinlaßloch (54) zum Liefern von Außenluft in dem Gasströmungsweg (26) zwischen der Vergasungseinrichtung (42) und der Saugöffnung (22) gebildet ist.

13. Aromaerzeugender Gegenstand nach einem der Ansprüche 1 bis 12, **gekennzeichnet durch** einen aus einem festen Material geformten Körper (92), welcher zumindest eine Aromasubstanz beinhaltet und in dem Gasströmungsweg (26) derart angeordnet ist, daß er zwischen der Vergasungseinrichtung (42) und der Saugöffnung (22) liegt.

14. Aromaerzeugender Gegenstand nach Anspruch 13, **gekennzeichnet durch** eine Heizeinrichtung (94) zum Erhitzen des geformten Körpers (92).

## Revendications

1. Article de génération d'arôme **caractérisé en ce qu'**il comporte :
un boîtier (12) ayant un orifice d'admission d'air (24) destiné à admettre de l'air dedans et un orifice d'aspiration (22) à travers lequel un utilisateur inhale un arôme, et formant un passage d'écoulement de gaz (26) entre ledit orifice d'admission et ledit orifice d'aspiration;
un réservoir de matière (32) destiné à emmagasiner une matière liquide qui contient au moins une substance d'arôme et ayant un orifice de sortie (35) pour ladite matière, ledit réservoir de matière étant monté sur ledit boîtier;
des moyens d'entraînement de refoulement (38) pouvant être actionnés électriquement afin de refouler ladite matière depuis ledit réservoir à travers ledit orifice de sortie sous la forme d'une goutte de liquide;
des moyens de gazéification (42) disposés dans ledit passage d'écoulement de gaz afin de recevoir la goutte de liquide de ladite matière refoulée depuis ledit réservoir par lesdits moyens d'entraînement de refoulement et de gazéifier ladite matière en chauffant électriquement la goutte de liquide;
une alimentation (62) destinée à délivrer de l'énergie électrique aux dits moyens d'entraînement de refoulement et aux dits moyens de gazéification; et
des moyens de commande (72) sensibles à l'aspiration appliquée sur l'orifice d'aspiration (22) afin de former de manière sélective un état dans lequel lesdits moyens d'entraînement de refoulement et lesdits moyens de gazéification fonctionnent en réponse à l'aspiration grâce à l'énergie électrique provenant de ladite alimentation.

2. Article de génération d'arôme selon la revendication 1, **caractérisé en ce qu'**il comporte en outre un capteur (73) destiné à détecter une opération d'inhalation de l'utilisateur, lesdits moyens de commande (72) commandant, sur la base d'un signal provenant dudit capteur, lesdits moyens d'entraînement de refoulement (38) et lesdits moyens de gazéification (42) de façon à refouler ladite matière depuis ledit réservoir de matière (32) et à générer de la chaleur grâce aux dits moyens de gazéification.

3. Article de génération d'arôme selon la revendication 2, **caractérisé en ce que** ledit capteur (73) comporte un capteur sensible à la pression monté sur ledit boîtier (12) autour dudit orifice d'aspiration (22).

4. Article de génération d'arôme selon la revendication 2 ou 3, **caractérisé en ce que** lesdits moyens de commande (72) commandent lesdits moyens d'entraînement de refoulement (38) et lesdits moyens de gazéification (42) sur la base du signal provenant dudit capteur (73) de telle sorte que lesdits moyens d'entraînement de refoulement et lesdits moyens de gazéification commencent à fonctionner en même temps.

5. Article de génération d'arôme selon la revendication 2 ou 3, **caractérisé en ce que** lesdits moyens de commande (72) commandent lesdits moyens de gazéification (42) et lesdits moyens d'entraînement de refoulement (38) sur la base du signal provenant dudit capteur (73) de façon à préchauffer lesdits moyens de gazéification avant de refouler ladite matière.

6. Article de génération d'arôme selon l'une quelconque des revendications 1 à 5, **caractérisé en ce que** lesdits moyens de commande (72) commandent lesdits moyens de gazéification (42) et lesdits moyens d'entraînement de refoulement (38) de telle sorte que lesdits moyens d'entraînement de refoulement et lesdits moyens de gazéification arrêtent de fonctionner en même temps.

7. Article de génération d'arôme selon la revendication 1, **caractérisé en ce que** ladite alimentation (62) est disposée dans ledit boîtier (12).

8. Article de génération d'arôme selon la revendication 7, **caractérisé en ce que** ledit boîtier (12) est constitué par des première et deuxième parties (12a, 12b) qui sont reliées électriquement l'une à l'autre par l'intermédiaire d'un câble (15), ledit passage d'écoulement de gaz (26), ledit réservoir (32), lesdits moyens d'entraînement de refoulement (38), lesdits moyens de gazéification (42) étant disposés dans ladite première partie (12a), et ladite alimentation (62) étant disposée dans ladite deuxième partie (12b).

9. Article de génération d'arôme selon la revendication 8, **caractérisé en ce que** lesdites première et deuxième parties (12a, 12b) dudit boîtier (12) sont reliées de façon démontable l'un à l'autre par l'intermédiaire d'une partie de raccordement (13).

10. Article de génération d'arôme selon l'une quelconque des revendications 1 à 9, **caractérisé en ce que** lesdits moyens de gazéification (42) comportent une couche poreuse (46), et la goutte de liquide de ladite matière est délivrée sur ladite couche poreuse.

11. Article de génération d'arôme selon l'une quelconque des revendications 1 à 10, **caractérisé en ce que** lesdits moyens de gazéification (42) sont disposés afin de faire face au dit orifice de sortie (35), et un trou d'étranglement (20) destiné à diriger un écoulement d'air depuis ledit orifice d'admission d'air (24) vers un espace entre ledit orifice de sortie et lesdits moyens de gazéification est disposé dans ledit passage d'écoulement de gaz (26).

12. Article de génération d'arôme selon l'une quelconque des revendications 1 à 11, **caractérisé en ce que** ledit boîtier (12) est formé avec un trou d'entrée d'air extérieur (54) afin de délivrer de l'air extérieur dans ledit passage d'écoulement de gaz (26) entre lesdits moyens de gazéification (42) et ledit orifice d'aspiration (22).

13. Article de génération d'arôme selon l'une quelconque des revendications 1 à 12, **caractérisé en ce qu'**il comporte en outre un corps formé (92) en matière solide contenant au moins une substance d'arôme et disposé dans ledit passage d'écoulement de gaz (26) de façon à être situé entre lesdits moyens de gazéification (42) et ledit orifice d'aspiration (22).

14. Article de génération d'arôme selon la revendication 13, **caractérisé en ce qu'**il comporte en outre des moyens de chauffage (94) destinés à chauffer ledit corps formé (92).
